# EUROPEAN PATENT SPECIFICATION

(11) **EP 1 932 480 B1**
(45) Date of publication and mention of the grant of the patent: **11.03.2015**
(21) Application number: 06425839.5
(22) Date of filing: 15.12.2006
(51) Int. Cl.: A61B 10/00

(54) **Support element for biopsy and kit for collecting and treating biopsies comprising this support**
Trägerelement für Biopsien und Satz zum Sammeln und Verarbeiten von Biopsien mit diesem Träger
Élément de support pour biopsie et kit pour la collecte et le traitement de biopsies comprenant ce support

(43) Date of publication of application: 18.06.2008
(73) Proprietor: Bio-Optica Milano S.p.A., 20134 Milano (IT)
(72) Inventor: Villanacci, Vincenzo, 25065 Concesio (Brescia) (IT)
(74) Representative: Croce, Valeria

(56) References cited:
- WO-A-00/76664
- WO-A-97/46864
- WO-A-97/49338
- GB-A- 501 873
- US-A- 2 278 339
- US-A- 3 552 929
- US-A- 5 031 764
- US-A1- 2005 142 031
- US-A1- 2006 064 035

## Description

### FIELD OF THE INVENTION

The present invention relates to a support element for biopsies and a kit for collecting and treating biopsies comprising this support. Particularly, the support and kit are such as to allow the biopsies to be easily and safely handled, thus ensuring an effective pathology diagnosis.

### BACKGROUND OF THE ART

The procedure of removing a sample of gastro-intestinal human tissue for diagnosis in a Pathological Anatomy laboratory is usually carried out by a doctor using conventional tools, such as endoscopes provided with forceps to cut small fragments of the gastro-intestinal mucosa and placing the latter into test tubes.

After or immediately after this removal, the samples or biopsies are dipped as such in fixative substances, such as formalin, and sent to said Pathological Anatomy Laboratory. In this laboratory, the biopsies are processed in accordance with routine procedures, which provide the fragments to be manipulated as such by being contained in containers and/or gauzes.

It is understood that biopsy fragments are handled such that when they are included on an inclusion box to prepare histological samples by means of microtome sections, the original arrangement of the layers composing the piece of tissue removed, or in other words, the proper orientation of the latter, is no longer identifiable.

The possibility of having "oriented" biopsies is thus left to chance for a good part, as the laboratory technician is forced to include the biopsies one by one by recovering the latter from the fixation medium, sometimes breaking them, without any reference point being available for orientation.

The proper orientation of the endoscopic biopsies of the gastro-intestinal tract, where "orientation" means the positioning of the biopsies to comply with the normal anatomic ratios between the various layers of the gastro-intestinal tract wall, is an essential prerequisite for a more accurate diagnostics of the various pathologic conditions.

The biopsy orientation is indeed very important for a pathologist for example for the diagnosis and grading of esophagitis, in the stomach for the differential diagnosis between superficial and atrophic gastritis, for the assessment of the grade of atrophy of the villi in the intestinal malabsorption disease, in the colon for the distinction between infectious colitis and idiopathic colitis, and within the idiopathic colitis for the differentiation between the Crohn disease and ulcerative rectocolitis.

To solve the problem of orientation, it has been proposed to use a support element for biopsies which consists of a piece or filter made of cellulose acetate or nitrocellulose on which the biopsies can be placed right after they have been removed.

This element is in the form of a checkered disc, which is manually cut in several strips of various lengths depending on the biopsy requirements and fixed on cork supports by means of pins. Care must be taken to mark an end of the strips in order to highlight the first biopsy.

At this stage, the biopsies are placed on each individual square along an individual straight line in order to allow all biopsies to be evenly cut. The thus obtained strip-biopsy assembly, may be placed in a container with the fixation medium for transportation to the Pathological Anatomy Laboratory without the risk that the biopsies may detach, as the strips allow a perfect adhesion to the tissue fragments.

During the subsequent inclusion step, the strip with the biopsies being fixed thereto is simply vertically included on a conventional microtome cutting box such that the biopsies are already in the proper position for cutting. In other words, the strip is lifted from the flat position to the vertical position such as to be placed on the box on one of the long sides thereof.

While the support described above overcomes the problems cited above with reference to the preparation of non-oriented biopsies, it is not without drawbacks.

Firstly, the support must be cut in rather small strips that, owing to the electrostatic nature of the material of which it is made, tend to adhere to the operator's hands rather than to the cutting tools or work surface.

Furthermore, after the strips have been obtained, they require to be fixed on the cork support usually by means of pins. This operation is also not easy, again, due to the size and electrostatic charge of the material.

A support as described above is thus quite uncomfortable and labour intensive to prepare.

In addition, the strips must be fixed on a support that conventionally consists of a cork piece. The cork, however, while offering a good support in the practice, does not allow the drainage of any liquid released from the biopsies, which may affect the adhesion of the biopsies and pollute the work surface.
WO 00176664 discloses a support element for biopsies as defined in the preamble of appended claim 1.

### SUMMARY OF THE INVENTION

The technical problem that lies at the heart of the present invention is thus to provide a support that overcomes all the drawbacks stated above.

This problem is solved by a support element that ensures the proper holding of a biopsy while resulting easy to use such as to promote a standardization of the procedure for preparing biopsies for anatomopathological analysis.

Accordingly, a first object of the present invention is to provide a support element for biopsies.

A second object is to provide a kit for collecting and treating biopsies comprising this support.

The invention is defined in appended independent claim 1. Preferred embodiments are described in the dependent claims.

### BRIEF DESCRIPTION OF THE FIGURES

Further characteristics and the advantages of the present invention will appear from the description below of an embodiment, which is given merely by way of nonlimiting example, with reference to the figures, in which:
- Fig. 1 is a plan view of a support element for biopsies according to the invention;
- Fig. 2 is a side view of the support element in Fig. 1.

### DETAILED DESCRIPTION OF THE INVENTION

The concept at the heart of the present invention is to reduce to a minimum the operations required for properly preparing the tissue samples for histological analysis, while avoiding conditions that may facilitate the contamination of biopsies.

It has been studied, accordingly, a support element that is ready to receive the biopsies and can be immediately transferred to the fixation step.

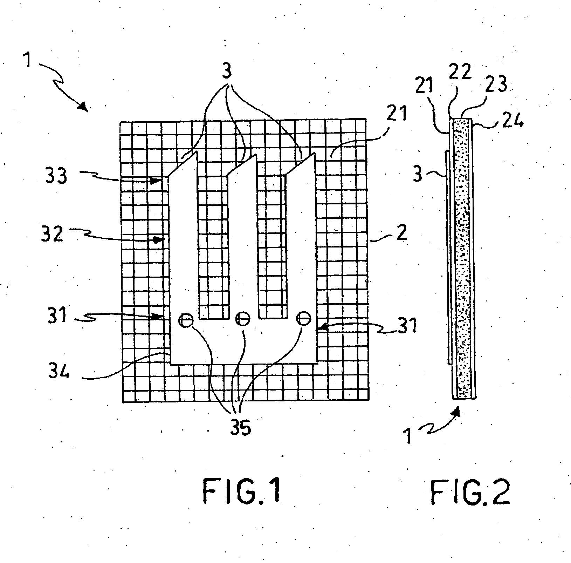

The support element 1, as shown in Fig. 1 and 2, comprises a substrate 2 to which at least one strip 3 of filtering material is adhered.

Particularly, the substrate 2 can be any piece of rigid, semi-rigid or soft material provided with a surface 21 in contact with said at least one strip 3 having filtering capacity. Preferably, this surface can be embodied by the free surface of a sheet 22 of resistant absorbent material, such as for example a sheet of absorbent paper fixed to said substrate 2.
Furthermore, the resistant sheet of absorbent material can be advantageously checkered, such as shown in Fig. 1, in order to provide a reference to the operator who is required to place the biopsy fragments on the support element.

The substrate material 2 can be a piece 23 of plastic, cork or wood, preferably extruded expanded polystyrene or similar elastically yielding soft material capable of holding any liquid that may pass through the sheet 22 of absorbent material. A particularly preferred material is the product sold under the trade name of LAMINIL^{®} 326.

The advantages obtained from selecting the above preferred material, in addition to the capacity of holding liquids, derive from the light-weight, the small thickness size, i.e. small bulk, the elasticity allowing such a deformability level as to facilitate operations subsequent to the deposit of biopsies, easy and cost-effective manufacture.

In addition, the substrate 2 can comprise a further sheet 24 of any paper material, preferably plasticized, that is applied to the surface of the substrate 2 opposite the surface 21 such as to create a sort of "sandwich-like structure". This second sheet can advantageously facilitate the manipulation of the whole support element 1 by imparting a greater rigidity degree whenever required by particular requirements or preferences for example dictated by an abundant absorption of liquids or a relatively wide surface of the support element relative to the thickness thereof.

In fact, as relates to the thickness, the support element 1 can be made with thickness values greater than about 1 mm, preferably ranging between 1,5 mm and 5 mm according to the type and amount of tissue sample to be removed and treated.

The piece 23 of material of the substrate 2 can be alternatively a filter as those that are normally used in a laboratory for carrying out filtrations of substances. Examples of these filters can be filters or membranes sold by Millipore® Company having a porosity ranging between 10 and 100 µm or similar materials.

The at least one strip 3 made of absorbent material has a general elongated rectangular shape and removably applied to the surface 21. The strip 3 comprises a first end 31 fixed to said surface, for example by means of gluing with bio-compatible glues, an elongated portion 32 and a second end 33 both being placed on said surface but not glued thereto. Preferably, the second end exhibits a sloped cut to indicate the position of the first biopsy.

Particularly, as shown in Fig. 1, the support element 1 comprises three strips 3 made of filtering material, which are arranged parallel to each other and spaced from each other. Preferably, the strips 3 are connected to each other at the first end 31 thereof, such as to form a belt 34 that is arranged substantially perpendicular to the strips.

Advantageously, the first ends 31 of the strips 3 have a weakening element 35 acting as a prompt for detaching the strips from the belt 34. This weakening element can be embodied, as in Fig. 1, by a circular interruption point or, alternatively, break lines that are typical, for example, in block notes paper sheets.

As described above, the material of the strips 3 is a filtering material, such as paper, cellulose acetate, cellulose nitrate, nylon, and other similar materials having the capacity of filtering and possibly also absorbing liquids and holding the biopsy samples on the surfaces thereof.

A material particularly suitable for this function is the cellulose nitrate or nitrocellulose, which in addition to absorbing the liquids, and thus performing a drainage function, has an electrostatically charged contact surface in order to ensure the adhesive capacity required to prevent the biopsies from becoming inadvertently detached. Furthermore, this material is easily tearable, such that the detachment of the strips 3 from the support element 1 results to be facilitated.

On the other hand, for conditions where a more resistant material is desirably selected, the use of nylon-based strips 3 results to be very advantageous due to the higher breaking strength thereof. Obviously, said weakening element 35 will be arranged such as to ensure the strips to be easily detached from the support element 1.

Materials like those described above are widely known for example in the field of biochemical research in technical laboratories and are commonly available on the market by manufacturers such as Osmonics GE, Sartorius, Woatman.

Preferably, the strips 3 have a size of 5 x 35 mm such as to fit conventional processing boxes for histological samples without forcing the technician to carry out further operations or cuts.

In addition, the surface 21 of the substrate 2, besides being checkered, is also coloured to highlight the strips 3, which are white.

The use of the support element 1 of the invention provides that right after the first removal, the biopsy is delicately laid on a strip 3 in the proximity of the sloped cut with the mucosa surface turned upwards and the submucosa downwards, i.e. the latter being rested on the strip.

Thereafter, the further removals are carried out and laid as above by arranging the biopsies along a straight line to the first end 31 of the strips 3 by following the checking on the surface 21 of substrate 2. This allows the technician to reduce the number of samples to be included and sections to be cut and coloured, as well as the pathologist to know the precise location of the biopsy site.

After the biopsy sampling has been carried out, the strip 3 is dipped in a fixative such as 10% neutral buffered formalin to fix the biopsies in accordance with fully conventional methods.

At this stage, the sample is ready to be included in suitable substances which allow the subsequent slicing by means of microtome.

In accordance with a further object of the present invention, a kit is provided for collecting and treating biopsies.

The kit comprises at least one support element 1 like that described above and at least one container for a fixative substance.

The container can be embodied by a conventional test tube having 4 ml capacity, provided with flat bottom and air-tight screw cap sufficient to contain a strip with biopsies completely dipped therein, thus reducing both the amount of formalin required for the fixation and the noxiousness of the formalin, which can be inadvertently inhaled by the operator.

Preferably, the kit consists of thirty test tubes pre-filled with formalin or other fixative and ten support elements each having three strips, which are individually packed preferably under openable sterile conditions.

Advantageously, the kit can comprise one or more means for gripping the strips in order to pick up the strips and tear them off the support element and place them in the suitable test tubes with formalin. These means can be embodied by disposable, preferably sterile forceps. It is understood that these operations can be carried out by reducing the contact with the biopsies to a minimum, thus avoiding pollutions that can alter the result of the analysis.

Further elements can be provided in the kit in accordance with the present invention, such as for example sterile gloves, pads for absorbing or cleaning the biopsies and/or the components of the support element from any liquid excess such as serum or blood.

Preferably, the kit comprises a leaflet carrying the instructions for use.

From what has been described above, the support element and kit of the invention have a number of advantages.

Particularly, the operating procedure of preliminary collecting and treating the biopsies can be standardized thus considerably facilitating the operators' work. Consequently, a considerable saving of time and material is ensured by avoiding the risk of breaking the prior art cellulose nitrate disc during the slicing just due to the provision of the support element.

Furthermore, with the standardization of the strip size, the width allows positioning the biopsies along an individual straight and ordered line, whereas the length is sufficient for the deposition of all the biopsies carried out during a same sampling. Thereby, after fixation in formalin, the strip can be contained in a conventional box for histological inclusions without being further resized and cut by the technician.

A sharp cut of the strip is also ensured simply by pulling it with forceps, due to the breaking element. In addition, the soft and collapsible substrate facilitates the grip with the forceps, considering that the strip tends to adhere to the substrate due to the characteristics discussed above. At the same time, the pins required for fixing the strips cut from the disc according to the prior art are avoided.

The risk that the operator may inhale the formalin fumes is then reduced, because the test tubes are not required to be filled, mainly in environments where an effective intake system cannot be obtained.

It should be noted, particularly, how the contamination risk between biopsy samples is completely eliminated due to the provision of a disposable product.

From what has been stated above, it is understood that the support element and the kit comprising this element are capable of meeting all sort of requirements and solve the technical problems exposed in the introductory part of the present description.

In any case, those skilled in the art, in order to meet particular requirements or preferences will be able of making further variations to the objects of the present invention without however departing from the scope of protection such as defined in the following claims.

For example, the number of strips 3 and the shape thereof can be changed to fit the size and type of the tissue being removed.

## Claims

1. A support element (1) for biopsies comprising a substrate (2) on which at least one strip (3) of filtering material is adhered, said substrate (2) being a piece of rigid, semi-rigid or soft material, which is provided with a surface (21) in contact with said at least one strip (3) of filtering material, wherein said at least one strip (3) of filtering material has a general elongated rectangular shape **characterized in that** the strip (3) of filtering material is removably applied to the surface (21) and comprises a first end (31) being fixed to said surface, an elongated portion (32) and a second end (33) both rested on said surface though not glued thereto, said at least one strip having a first end having a weakening element (35) arranged such as to ensure the strip to be easily detached from the support element.

2. The support element (1) according to claim 1, wherein said surface (21) is embodied by the free surface of a sheet (22) made of a tear-resistant absorbent material.

3. The support element (1) according to claim 2, wherein said sheet (22) of absorbent material is checkered.

4. The support element (1) according to any claim 1 to 3, wherein said piece of substrate material (2) comprises a piece (23) of plastic, cork, wood, extruded expanded polystyrene, or other elastically yielding material similar thereto which is capable of holding liquids or a filter or membrane used for filtering substances.

5. The support element (1) according to claim 4, wherein said piece (23) is extruded expanded polystyrene sold under the name of LAMINIL 326.

6. The support element (1) according to any claim 1 to 5, wherein the substrate (2) further comprises a further sheet of paper material preferably plasticized that is applied on the surface thereof opposite the surface (21).

7. The support element (1) according to any claim 1 to 6, having a thickness higher than 1 mm, preferably ranging between 1,5 and 5 mm.

8. The support element (1) according to claim 1, wherein said second end shows a sloped cut.

9. The support element (1) according to any claim 1 to 8, wherein said at least one strip (3) is formed by a filtering material such as paper, cellulose acetate, cellulose nitrate, nylon and other simitar materials having the capacity of absorbing liquids and holding the biopsies on the surface thereof.

10. The support element (1) according to any claim 1 to 9, wherein said at least one strip (3) has a size of 5 x 35 mm.

11. A kit for collecting and treating biopsies comprising at least one support element (1) according to any preceding claim and at least one container for a fixative substance.

12. The kit according to claim 11, wherein said container is a test tube having 4 ml capacity, provided with screw cap and flat bottom.

13. The kit according to claim 11 or 12, further comprising one or more means for gripping the strips such as forceps and/or sterile gloves and/or pads for absorbing or cleaning the biopsies or the support element (1).

14. The kit according to any claim 11 to 13, comprising several support elements (1) that are sealed in a sterile manner and a leaflet indicating the instructions for using said kit.

## Patentansprüche

1. Trägerelement (1) für Biopsien, umfassend ein Substrat (2), auf welchem mindestens ein Streifen (3) aus Filtermaterial angehaftet ist, wobei das Substrat (2) ein Teil aus rigidem, semirigidem oder weichem Material ist, welches mit einer Oberfläche (21) in Kontakt mit dem mindestens einen Streifen (3) aus Filtermaterial bereitgestellt ist, wobei der mindestens eine Streifen (3) aus Filtermaterial eine allgemeine gestreckte rechtwinkelige Form aufweist, **dadurch gekennzeichnet, dass** der Streifen (3) aus Filtermaterial entfernbar auf die Oberfläche (21) aufgebracht ist, und ein erstes Ende (31), das an die Oberfläche fixiert ist, einen gestreckten Teil (32) und ein zweites Ende (33), beide auf der Oberfläche aufliegend jedoch nicht daran geklebt, umfasst, wobei der mindestens eine Streifen ein erstes Ende mit einem Schwächungselement (35) aufweist, das so angeordnet ist, um beispielsweise sicherzugehen, dass der Streifen einfach von dem Trägerelement abgetrennt wird.

2. Trägerelement (1) nach Anspruch 1, wobei die Oberfläche (21) durch die freie Oberfläche einer Folie (22), aus reißfestem Absorbensmaterial gemacht, verkörpert wird.

3. Trägerelement (1) nach Anspruch 2, wobei die Folie (22) aus Absorbensmaterial kariert ist.

4. Trägerelement (1) nach einem Anspruch 1 bis 3, wobei das Teil aus Substratmaterial (2) ein Teil (23) aus Plastik, Kork, Holz, extrudiertem expandiertem Polystyrol oder anderem elastisch nachgebendem Material ähnlich dazu, welches in der Lage ist Flüssigkeiten zu halten, oder einem Filter oder Membran, das zum Filtrieren von Substanzen verwendet wird, umfasst.

5. Trägerelement (1) nach Anspruch 4, wobei das Teil (23) extrudiertes expandiertes Polystyrol ist, das unter dem Namen LAMINIL 326 verkauft wird.

6. Trägerelement (1) nach einem Anspruch 1 bis 5, wobei das Substrat (2) weiter eine weitere Folie aus Papiermaterial, bevorzugt plastifiziert, umfasst, das auf der Oberfläche davon, gegenüber der Oberfläche (21), aufgebracht ist.

7. Trägerelement (1) nach einem Anspruch 1 bis 6, mit einer Dicke höher als 1 mm, bevorzugt sich zwischen 1,5 und 5 mm erstreckend.

8. Trägerelement (1) nach Anspruch 1, wobei das zweite Ende einen schrägen Schnitt zeigt.

9. Trägerelement (1) nach einem Anspruch 1 bis 8, wobei der mindestens eine Streifen (3) gebildet wird durch ein Filtermaterial, wie Papier, Celluloseacetat, Cellulosenitrat, Nylon und andere ähnliche Materialien, mit der Eigenschaft Flüssigkeiten zu absorbieren und die Biopsien auf der Oberfläche davon zu halten.

10. Trägerelement (1) nach einem Anspruch 1 bis 9, wobei der mindestens eine Streifen (3) eine Größe von 5 x 35 mm aufweist.

11. Kit zum Sammeln und Behandeln von Biopsien, umfassend mindestens ein Trägerelement (1) nach einem vorhergehenden Anspruch und mindestens ein Behälter für eine Fixiermittelsubstanz.

12. Kit nach Anspruch 11, wobei der Behälter ein Reagenzröhrchen mit 4 mL Kapazität ist, der mit Schraubdeckel und flacher Unterseite bereitgestellt wird.

13. Kit nach Anspruch 11 oder 12, weiter umfassend ein oder mehrere Mittel zum Greifen der Streifen, wie beispielsweise Zange und/oder sterile Handschuhe und/oder Pads zum Absorbieren oder Reinigen der Biopsien oder des Trägerelements (1).

14. Kit nach einem Anspruch 11 bis 13, umfassend mehrere Trägerelemente (1), die auf eine sterile Art versiegelt sind, und einer Druckschrift, die die Anweisung zur Verwendung des Kits angibt.

## Revendications

1. Elément de support (1) pour biopsies comprenant un substrat (2) sur lequel au moins une bande (3) de matériau filtrant est amenée à adhérer, ledit substrat (2) étant un fragment de matériau rigide, semi-rigide ou mou, qui est muni d'une surface (21) en contact avec ladite au moins une bande (3) de matériau filtrant, où ladite au moins une bande (3) de matériau filtrant a une forme générale rectangulaire allongée **caractérisé en ce que** la bande (3) de matériau filtrant est appliquée de manière amovible à la surface (21) et comprend une première extrémité (31) qui est fixée à ladite surface, une partie allongée (32) et une seconde extrémité (33) l'une et l'autre reposant sur ladite surface bien que n'étant pas collées à celle-ci, ladite au moins une bande ayant une première extrémité ayant un élément d'affaiblissement (35) disposé de manière à garantir que la bande soit détachée aisément de l'élément de support.

2. Elément de support (1) selon la revendication 1, où ladite surface (21) est concrétisée par la surface libre d'une feuille (22) faite d'un matériau absorbant résistant à la déchirure.

3. Elément de support (1) selon la revendication 2, où ladite feuille (22) de matériau absorbant est quadrillé.

4. Elément de support (1) selon l'une quelconque des revendications 1 à 3, où ledit fragment de matériau de substrat (2) comprend un fragment (23) de plastique, de liège, de bois, de polystyrène expansé extrudé ou d'un autre matériau élastique similaire à ceux-ci qui est capable de maintenir des liquides ou un filtre ou une membrane utilisée pour filtrer des substances.

5. Elément de support (1) selon la revendication 4, où ledit fragment (23) est du polystyrène expansé extrudé commercialisé sous la dénomination de LAMINIL 326.

6. Elément de support (1) selon l'une quelconque des revendications 1 à 5, où le substrat (2) comprend en outre une autre feuille de matériau de type papier de préférence plastifié qui est appliquée sur sa surface opposée à la surface (21).

7. Elément de support (1) selon l'une quelconque des revendications 1 à 6 ayant une épaisseur supérieure à 1 mm, de préférence située entre 1,5 et 5 mm.

8. Elément de support (1) selon la revendication 1, où ladite seconde extrémité présente une découpure en biais.

9. Elément de support (1) selon l'une quelconque des revendications 1 à 8, où ladite au moins une bande (3) est formée par un matériau filtrant comme le papier, l'acétate de cellulose, le nitrate de cellulose, le Nylon et d'autres matériaux similaires ayant la capacité d'absorber les liquides et de maintenir les biopsies sur leur surface.

10. Elément de support (1) selon l'une quelconque des revendications 1 à 9, où ladite au moins une bande (3) a une taille de 5 x 35 mm.

11. Kit pour recueillir et traiter des biopsies comprenant au moins un élément de support (1) selon l'une quelconque des revendications précédentes et au moins un récipient pour une substance fixatrice.

12. Kit selon la revendication 11, où ledit récipient est un tube à essai ayant une capacité de 4 ml, muni d'un bouchon à vis et d'un fond plat.

13. Kit selon la revendication 11 ou 12 comprenant en outre un ou plusieurs moyens pour saisir les bandes comme une pince et/ou des gants stériles et/ou des tampons pour absorber ou nettoyer les biopsies ou l'élément de support (1).

14. Kit selon l'une quelconque des revendications 11 à 13 comprenant plusieurs éléments de support (1) qui sont scellés d'une manière stérile et un feuillet indiquant les instructions pour utiliser ledit kit.
